# EUROPEAN PATENT SPECIFICATION

(11) **EP 2 563 304 B1**
(45) Date of publication and mention of the grant of the patent: **09.11.2016**
(21) Application number: 11723277.7
(22) Date of filing: 23.03.2011
(51) Int. Cl.: A61F 11/08, A01K 15/02, A61K 9/00

(54) **METHODS FOR REVERSIBLY REDUCING THE AURAL SENSITIVITY OF ANIMALS**
VERFAHREN FÜR DIE REVERSIBLE VERRINGERUNG DER GEHÖREMPFINDLICHKEIT BEI TIEREN
PROCÉDÉS POUR LA RÉDUCTION RÉVERSIBLE DE LA SENSIBILITÉ ACOUSTIQUE DES ANIMAUX

(30) Priority: 27.04.2010 GB 201007011
(43) Date of publication of application: 06.03.2013
(73) Proprietor: Chamberlain, David, Guernsey GY7 9AL (GB)
(72) Inventor: Chamberlain, David, Guernsey GY7 9AL (GB)
(74) Representative: Muir, Justine Claire
(86) International application number: PCT/EP2011/054473
(87) International publication number: WO 2011/134722

(56) References cited:
- WO-A2-2005/055921
- US-A1- 2005 244 342
- US-A1- 2006 034 779
- US-A1- 2008 138 296
- US-B1- 7 506 720
- CYNTHIA EDGERLY: 'FIDO'S FEAR OF FIREWORKS & OTHER LOUD NOISES' BINGO! DOG TRAINING...WHERE THE DOGS & THE PEOPLE WIN, [Online] Retrieved from the Internet: <URL:https://web.archive.org/web/2008050304 2016/http://www.bingodogtraining.com/SoundS ensitivity.htm> [retrieved on 2015-05-28]

## Description

The invention relates to methods of using acoustic dampening fluid compositions for reversibly, reducing the sensitivity of animals to sounds such as fireworks to alleviate phobias in animals.

Phobic behaviour in response to sounds such as fireworks is exhibited by large numbers of domestic animals. Phobic behaviours caused by a hypersensitivity to sounds includes by way of example only: barking for extended periods in dogs triggered by noise; dogs who's travel stress is triggered and maintained by certain noises; and extreme fear in horses, dogs and cats in response to certain sounds especially loud and unpredictable sounds such as those produced by fireworks. Currently there is no treatment for this distressing condition even though it affects large numbers of pets and their owners. A need exists for a means of treating hypersensitivity to sounds in sound phobic animals.

The present invention provides a safe and reversible treatment for sound phobic animals comprising an acoustic dampening fluid composition in the form of foams, which "dampen" hearing sufficiently to reduce sound hypersensitivity symptoms in sound phobic animals.

In a first aspect the invention relates to a method of temporarily impairing the hearing of a non-human animal comprising applying an acoustic dampening fluid composition comprising: an oily or non-aqueous substance, an inert solvent and a surfactant to the external ear canal of the non-human animal.

In a second aspect the invention relates to a method of desensitising non-human animals to sounds comprising the following steps:
a) applying an acoustic dampening fluid composition according to the method claimed in claim 1;
b) exposing the animal to the offensive sound or sounds until some tolerance to the sound or sounds has been established ;
c) allowing or causing the first acoustic dampening fluid to be dispersed from the ears;
d) applying a second acoustic dampening fluid composition having decreased acoustic absorptive and/or dampening properties to the ears;
e) and repeating the exposure to the offensive sounds until tolerance to the sound has been re-established .

Preferred features of the methods are defined in claims 3 to 7.

### Detailed description:

According to the present invention an acoustic dampening fluid composition is applied to the external ear canal to temporarily impair hearing and reduce sensitivity to sound.

The acoustic dampening fluid composition is applied to the external ear canal. The composition fills both the external ear canal and covers the ear drum. This both dampens vibration of the ear drum and absorbs sound waves as they enter the ear canal.

The acoustic dampening fluid composition is inert, and non-irritant. The acoustic dampening fluid composition comprises an oily or non-aqueous component. Preferably the oily or non-aqueous substance is present in an amount of 10,000 mg/kg - 300,000 mg/kg, more preferably 50,000 mg/kg - 200,000 mg/kg.

The acoustic dampening fluid composition further comprises a suitable solvent such as by way of example water, lower alcohols, glycols and combinations thereof.

Preferably the dampening fluid compositions according to the present invention are provided as a metered aerosol, a single-shot vial, or as a two-compartmental system such as a "bag-in-can" type aerosol product. Packaging in a pressurized container is desirable to mitigate against premature expansion of the mousse. However the mechanism which delivers the foam or mousse into the external ear canal must be silent so as not to alarm or distress the animal or human.

Preferably the acoustic dampening fluid remains in the ear for a short period ranging from between a few hours and a few days where it causes a temporary period of impaired hearing. More preferably the acoustic dampening fluid remains in the ear for 3 hours before beginning to disperse.

Thereafter the acoustic dampening fluid degrades and/or disperses from the ear drum and ear canal returning the hearing to its former normal level of sensitivity.

In the unlikely event of an adverse or allergic reaction being provoked by the method of the present invention the foam can be rapidly dispersed using an inert, non-irritant solvent.

Preferably the acoustic dampening fluid composition further comprises one or more of: a smart liquid or gel, a smart emulsion, an expanding foam and a non-expanding foam. Smart materials as used herein are materials which have one or more properties that can be significantly changed in a controlled manner in response to external stimuli. By way of example only post-foaming compositions are liquids or gels in their packaging which alter their state and become for example a foam or mousse once exposed to any of air, moisture, release from a pressurised container or mixing of multiple components together.

Optionally the acoustic dampening fluid composition further comprises one or more thickeners.

Especially preferred thickeners may be selected from: povidone, cellulose gums such as sodium carmellose, xanthan gum and other pharmaceutically acceptable thickeners, or combinations thereof.

These thickeners may also act as stabilisers for the composition and help it slowly develop from a gel or liquid to a foam or mousse after application to the external ear canal, as well as improving the mucoadhesive and skin adhesive qualities of the formulation.

Preferably thickeners are present in an amount of 1000 mg/kg - 300,000 mg/kg, more preferably 10,000 mg/kg - 150,000 mg/kg.

The acoustic dampening fluid composition further comprises one or more surfactants which assist in the formation of the foam or mousse, the emulsification of the vapour pressure reducers, and the solubilisation of the active agents. Especially preferred surfactants may be selected from nonoxinol, polysorbate, glyceryl stearate, glycol stearate, sorbitan esters, polyoxylated stearates, polyoxylated castor oil, macrogol alkyl ethers such as macrogol lauryl ether, and other pharmaceutically acceptable surfactants or combinations thereof.

Preferably surfactants are present in an amount of 10,000 mg/kg - 600,000 mg/kg , more preferably 40,000 mg/kg - 400,000 mg/kg.

Optionally the acoustic dampening fluid composition may further comprises one or more vapour pressure reducers. Vapour pressure reducers are often oily substances such as for example caprylic/capric triglycerides. They may act as solvents or be used in conjunction with foaming agents.

Optionally the acoustic dampening fluid composition may further comprises one or more excipient such as by way of example only pH buffers, solvents, carriers, diluents and preservatives. Suitable pH buffers may include citrates, phosphates or acetates, or other pharmaceutically acceptable buffers. Suitable preservatives may include benzyl alcohol or parabens, and other pharmaceutically acceptable preservatives. Suitable carriers and diluents may include purified water.

Preferably pH buffers are present in an amount of 0 - 100,000 mg/kg, more preferably 1000 mg/kg - 200,000 mg/kg.

Preferably preservatives are present in an amount of 100 mg/kg - 50,000 mg/kg, more preferably 200 mg/kg - 20,000 mg/kg.

By adjusting the various constituents of the dampening fluid composition the skilled addressee can adjust a number of its characteristics for example:
- viscosity
- adhesion to skin of the external ear canal
- duration of persistence of the foam or mousse
- nature of the spent foam or exhausted components
- how easy spent foam is removed from the external ear canal by the body's natural mechanisms
- if a "cleaner" is required to remove the spent foam.

Dampening fluid compositions according to the invention may be prepared by blending all components into the preferred solvent using an overhead stirrer. Alternative mixing protocols are also envisaged such as by way of example only using two cylinders with pistons connected to receiving compartments for the various components of the dampening fluid. The pistons share a shaft and cylinders connect via a conduit. As the pistons cycle the components of the compound are mixed.

In preferred embodiment the acoustic dampening fluid composition is provided as a post foaming composition.

In an especially preferred embodiment the acoustic dampening fluid composition is provided as liquid or gel at room temperature which is easily administered to the ear canal.

Shortly thereafter as a result of the inclusion of foaming agents in the composition the fluid slowly foams at a rate which neither alarms or distresses the animal to a degree sufficient to adequately coat the skin surface within the external ear canal so as to provide a physical barrier to noise.

Foaming agents are pharmaceutically acceptable propellants. Suitable foaming agents may be selected from hydrocarbons such as n-butane, chlorofluorocarbons, hydrofluorocarbons, dimethyl ether, nitrogen, nitrous oxide, oxygen, ozone and carbon dioxide.

The acoustic dampening fluid composition must be formulated so that the foaming agent does not cause excessively rapid expansion of the foam in the external ear canal of an animal since discomfort and/or pain will result.

Once the foam collapses its spent components are small in volume and are quickly and easily cleared by the ears natural mechanisms to effect dispersal of the acoustic dampening fluid. If necessary, or desired a solvent or cleaner can be applied to accelerate removal of the spent foam.

The safety, ease of use and reversibility of the method of the present invention makes it ideally suited to the treatment of sound phobias in.

The acoustic dampening fluid in the form of foam composition provides an alternative to conventional ear plugs and ear protectors which are impractical in animals and cumbersome and unsightly for humans.

The invention also relates to a method of progressively desensitising animals to sounds to which they are hypersensitive. The method involves applying a first acoustic dampening fluid composition to the ears of an affected animal and exposing the animal to the offensive sound or sounds. according to the claims.

Then once some tolerance to the sound or sounds has been established dispersing the first acoustic dampening fluid composition from the ears and applying a second acoustic dampening fluid composition having decreased acoustic absorptive/dampening properties to the ears and repeating the exposure to the offensive sounds until tolerance to the sound has been re-established . This is a classical desensitisation protocol for desensitising individuals to a stimulus to which they have a phobia.

Preferably the second acoustic dampening fluid composition is obtained by diluting the first acoustic dampening fluid with a suitable solvent.

## Claims

1. A method of temporarily impairing the hearing of a non-human animal comprising applying an acoustic dampening fluid composition comprising: an oily or non-aqueous substance, an inert solvent and a surfactant to the external ear canal of the non-human animal.

2. A method of desensitising non-human animals to sounds comprising the following steps:
a) applying an acoustic dampening fluid composition according to the method claimed in claim 1;
b) exposing the animal to the offensive sound or sounds until some tolerance to the sound or sounds has been established ;
c) allowing or causing the first acoustic dampening fluid to be dispersed from the ears;
d) applying a second acoustic dampening fluid composition having decreased acoustic absorptive and/or dampening properties to the ears;
e) and repeating the exposure to the offensive sounds until tolerance to the sound has been re-established .

3. A method as claimed in claim 1 or claim 2 wherein the oily or non-aqueous substance is present in an amount of 10,000 mg/kg - 300,000 mg/kg of the acoustic dampening fluid composition.

4. A method as claimed in any preceding claim wherein the oily or non-aqueous substance is present in an amount of 50,000 mg/kg - 200,000 mg/kg of the acoustic dampening fluid composition.

5. A method as claimed in any preceding claim wherein the acoustic dampening fluid composition further comprises one or more of: a thickener, a smart liquid, a smart gel, a smart emulsion, foaming agents and vapour pressure reducers.

6. A method as claimed in any preceding claim wherein the acoustic dampening fluid composition further comprises one or more excipient selected from pH buffers, solvents, carriers, diluents and preservatives.

7. A method as claimed in any preceding claim wherein the non-human animal is a cat, a dog, a horse, a rabbit or like domestic animal.

## Patentansprüche

1. Verfahren zum vorübergehenden Beeinträchtigen des Gehörs eines Tieres, das das Auftragen einer Schalldämpfungsfluidzusammensetzung auf den äußeren Gehörgang des Tieres umfasst, wobei die Zusammensetzung Folgendes umfasst: eine ölige oder nicht-wässrige Substanz, ein inertes Lösungsmittel und ein Tensid.

2. Verfahren zur Desensibilisierung von Tieren gegen Geräusche, wobei das Verfahren folgende Schritte umfasst:
a) Auftragen einer Schalldämpfungsfluidzusammensetzung gemäß dem Verfahren nach Anspruch 1;
b) Aussetzen des Tieres gegenüber dem offensiven Geräusch bzw. den Geräuschen, bis eine gewisse Toleranz gegenüber dem Geräusch bzw. den Geräuschen festgestellt wurde;
c) Zulassen oder Veranlassen, dass das erste Schalldämpfungsfluid aus den Ohren dispergiert wird;
d) Auftragen einer zweiten Schalldämpfungsfluidzusammensetzung, die verringerte Schallabsorptions- und/oder Dämpfungseigenschaften für die Ohren aufweist;
e) und Wiederholen der Aussetzung gegenüber den offensiven Geräuschen, bis die Toleranz gegenüber dem Geräusch wiederhergestellt wurde.

3. Verfahren nach Anspruch 1 oder Anspruch 2, wobei die ölige oder nicht-wässrige Substanz in einer Menge von 10.000 mg/kg bis 300.000 mg/kg der Schalldämpfungsfluidzusammensetzung vorliegt.

4. Verfahren nach einem der vorhergehenden Ansprüche, wobei die ölige oder nicht-wässrige Substanz in einer Menge von 50.000 mg/kg bis 200.000 mg/kg der Schalldämpfungsfluidzusammensetzung vorliegt.

5. Verfahren nach einem der vorhergehenden Ansprüche, wobei die Schalldämpfungsfluidzusammensetzung ferner eines oder mehrere von Folgendem umfasst: ein Verdickungsmittel, eine intelligente Flüssigkeit, ein intelligentes Gel, eine intelligente Emulsion, Schäumungsmittel und Dampfdruckminderer.

6. Verfahren nach einem der vorhergehenden Ansprüche, wobei die Schalldämpfungsfluidzusammensetzung ferner einen oder mehrere Exzipienten umfasst, die aus Folgendem ausgewählt werden: pH-Puffer, Lösungsmittel, Träger, Verdünnungsmittel und Konservierungsmittel.

7. Verfahren nach einem der vorhergehenden Ansprüche, wobei das Tier eine Katze, ein Hund, ein Pferd, ein Kaninchen oder ein ähnliches Haustier ist.

## Revendications

1. Procédé pour affaiblir temporairement l'ouïe d'un animal non humain comprenant l'étape consistant à appliquer une composition fluide d'amortissement acoustique comprenant : une substance huileuse ou non aqueuse, un solvant inerte et un tensioactif dans le conduit auditif externe de l'animal non humain.

2. Procédé pour désensibiliser des animaux non humains à des sons comprenant les étapes suivantes consistant à :
a) appliquer une composition fluide d'amortissement acoustique selon le procédé revendiqué dans la revendication 1 ;
b) exposer l'animal au son ou aux sons déplaisants jusqu'à l'établissement d'une certaine tolérance au son ou aux sons ;
c) laisser ou amener le premier fluide d'amortissement acoustique à se disperser des oreilles ;
d) appliquer une deuxième composition fluide d'amortissement acoustique ayant des propriétés d'absorption et/ou d'amortissement acoustiques réduites dans les oreilles ;
e) répéter l'exposition aux sons déplaisants jusqu'au rétablissement de la tolérance au son.

3. Procédé selon la revendication 1 ou la revendication 2, dans lequel la substance huileuse ou non aqueuse est présente dans une quantité de 10 000 mg/kg à 300 000 mg/kg de la composition fluide d'amortissement acoustique.

4. Procédé selon l'une quelconque des revendications précédentes dans lequel la substance huileuse ou non aqueuse est présente dans une quantité de 50 000 mg/kg à 200 000 mg/kg de la composition fluide d'amortissement acoustique.

5. Procédé selon l'une quelconque des revendications précédentes dans lequel la composition fluide d'amortissement acoustique comprend en outre un ou plusieurs excipients choisis dans le groupe comprenant : un épaississant, un liquide intelligent, un gel intelligent, une émulsion intelligente, des agents moussants et des réducteurs de la pression de vapeur.

6. Procédé selon l'une quelconque des revendications précédentes dans lequel la composition fluide d'amortissement acoustique comprend en outre un ou plusieurs excipients choisis dans le groupe comprenant des tampons de pH, des solvants, des supports, des diluants et des conservateurs.

7. Procédé selon l'une quelconque des revendications précédentes dans lequel l'animal non humain est un chat, un chien, un cheval, un lapin ou un animal domestique similaire.
